# EUROPEAN PATENT SPECIFICATION

(11) **EP 3 173 488 B1**
(45) Date of publication and mention of the grant of the patent: **09.09.2020**
(21) Application number: 15824842.7
(22) Date of filing: 23.07.2015
(51) Int. Cl.: C12Q 1/68, C12Q 1/6858

(54) **USE OF MULTIPLE TARGET NUCLEIC ACID DETECTION METHOD USING CLAMPING PROBE AND DETECTION PROBE**
VERWENDUNG VON DETEKTIONSVERFAHREN FÜR MEHRFACHZIEL-NUKLEINSÄUREN UNTER VERWENDUNG VON KLEMMSONDE UND DETEKTIONSSONDE
UTILISATION DE MULTIPLES PROCÉDÉS DE DÉTECTION D'ACIDE NUCLÉIQUE CIBLE ET SONDE DE DÉTECTION À L'AIDE DE SONDE DE SERRAGE

(30) Priority: 25.07.2014 KR 20140095050
(43) Date of publication of application: 31.05.2017
(73) Proprietor: Panagene Inc., Daejeon 305-510 (KR)
(72) Inventor: CHOI, Jae Jin, Daejeon 305-793 (KR); YOON, Ji Hye, Anseong-si Gyeonggi-do 456-783 (KR); KIM, Su Nam, Daejeon 305-509 (KR); KIM, Sung Kee, Daejeon 305-500 (KR); KIM, Jin Woo, Daejeon 305-722 (KR)
(74) Representative: Huenges, Martin
(86) International application number: PCT/KR2015/007656
(87) International publication number: WO 2016/013880

(56) References cited:
- WO-A1-2008/009740
- WO-A1-2015/068957
- WO-A2-2011/105732
- KR-A- 20150 054 633
- US-A1- 2008 176 226
- NAGAI YOSHIAKI ET AL: "Genetic heterogeneity of the epidermal growth factor receptor in non-small cell lung cancer cell lines revealed by a rapid and sensitive detection system, the peptide nucleic acid-locked nucleic acid PCR clamp", CANCER RESEARCH, vol. 65, no. 16, 1 August 2005 (2005-08-01), pages 7276-7282, XP002413948, AACR - AMERICAN ASSOCIATION FOR CANCER RESEARCH, US ISSN: 0008-5472, DOI: 10.1158/0008-5472.CAN-05-0331
- YOONJUNG KIM ET AL: "Spectrum of EGFR Gene Copy Number Changes and KRAS Gene Mutation Status in Korean Triple Negative Breast Cancer Patients", PLOS ONE, vol. 8, no. 10, E79014, 30 October 2013 (2013-10-30), pages 1-7, XP055458087, DOI: 10.1371/journal.pone.0079014
- Panagene: "EGFR/KRAS/BRAF Mutation Detection Kit", GEN , vol. 30, no. 16 15 September 2010 (2010-09-15), XP002778991, Retrieved from the Internet: URL:https://www.genengnews.com/new-product s/egfrkrasbraf-mutation-detection-kit/3597 [retrieved on 2018-03-12]
- CRISTINA GARCÍA-INCLÁN ET AL: "EGFR status and KRAS/BRAF mutations in intestinal-type sinonasal adenocarcinomas", CELLULAR ONCOLOGY, vol. 35, no. 6, 11 October 2012 (2012-10-11), pages 443-450, XP055458403, Dordrecht ISSN: 2211-3428, DOI: 10.1007/s13402-012-0103-7
- OH ET AL.: 'Detection of low-level KRAS mutations using PNA-mediated asymmetric PCR clamping and melting curve analysis with unlabeled probes' THE JOURNAL OF MOLECULAR DIAGNOSTICS vol. 12, no. 4, 01 July 2010, pages 418 - 424, XP055256008 DOI: 10.2353/JMOLDX.2010.090146
- EL KADER ET AL.: 'The KRAS StripAssay for detection of KRAS mutation in Egyptian patients with colorectal cancer (CRC): A pilot study' JOURNAL OF THE EGYPTIAN NATIONAL CANCER INSTITUTE vol. 25, no. 1, 01 March 2013, pages 37 - 41, XP055389243 DOI: 10.1016/J.JNCI.2012.12.003
- CHEN ET AL.: 'Rapid detection of K-ras mutations in bile by peptide nucleic acid-mediated PCR clamping and melting curve analysis: comparison with restriction fragment length polymorphism analysis' CLINICAL CHEMISTRY vol. 50, no. 3, 01 March 2004, pages 481 - 489, XP055064603 DOI: 10.1373/CLINCHEM.2003.024505
- MARCHETTI ET AL.: 'Clinical implications of KRAS mutations in lung cancer patients treated with tyrosine kinase inhibitors: an important role for mutations in minor clones' NEOPLASIA vol. 11, no. 10, 01 October 2009, pages 1084 - 1092, XP055389246 DOI: 10.1593/NEO.09814

## Description

### TECHNICAL FIELD

The present invention relates to a method for detecting a target nucleic acid using a clamping probe and a detection probe, and the use thereof. As the method can detect a small amount of mutations contained in a gene sample rapidly and sensitively, the present invention can be used for various purposes. In particular, the present invention can provide gene mutation detection methods and various information necessary for diagnosis or studies of diseases associated with gene mutations.

In addition, the present invention relates to a method for supporting diagnosis of a disease or a medical condition, prognosis, monitoring of a disease, treatment efficacy evaluation, nucleic acid and protein delivery studies and so on, by sensitively detecting a small amount of mutations contained in a gene. Further, the present invention relates to a method for monitoring the progress of various diseases (e.g. cancer) in a subject and a method for monitoring recurrence.

### BACKGROUND ART

By conducting a genetic test, a genetic disease suspected in a patient who has a symptom of the disease can be confirmed or identified.
MH:gb

Further, through the test for the presence of mutation of a gene which causes a disorder, it is also possible to predict the likelihood of a disease, and predict disorders having higher risk of occurrence. It has been reported that such genetic tests are helpful in the prevention, early diagnosis, treatment and management of a disease, which results in reducing the morbidity rate and the mortality of the disorder.

Such genetic tests can be conducted with any tissues of a human body, based on the premise that all cells in the human body are composed genetically in the same manner. More easily, the genetic tests can be conducted by using DNA extracted from the blood. Examples of various samples include DNA, RNA, chromosomes, metabolites, and the like.

In particular, as the fact that abnormalities of genes, such as mutations of oncogenes, mutations of tumor suppressor genes, deregulations, chromosomal abnormalities, etc., are involved in the determination of occurrence of a cancer and prognosis of a drug has been discovered, various studies have been reported (Fearson ER et al., Cell., 1990; K.W Kinzler et al., Cell., 1996; Manuel Serrano et al., Cell., 1997; Amado RG et al., J. Clin. Oncol., 2008, Raponi M et al., Curr. Opin, Pharmacol., 2008; Siena S et al., J. Natl.Cancer Inst., 2009).

By analysis of genes specific to a cancer, the presence of occurrence of cancer can be indirectly confirmed. For example, there are a relative increase of EGFR nucleic acid, and a relative decrease of tumor suppressor genes such as p53, etc.

In addition, the reports on the association between the function of oncogenes and cancers, and drug reactivity, etc., have suggested the need of tests for mutations of oncogenes. For example, there are reports that when cetuximab, which is a therapeutic agent of an anti-EGFR antibody, was administered to a normal KRAS patient after conducting the test for mutations of KRAS exon 2 (codons 12, 13) gene which is commonly observed in colorectal cancers, the reaction rate was 35 to 45% (Lievre et al. Cancer Res 2006; 66:3992-3995), and in the progress of the study for identifying different mutant types of RAS, the mutation rate of KRAS exon 2 (codons 12, 13) was 40 to 45%, and in case where KRAS exon 2 is normal, the mutation rates of KRAS codons 61, 146 and NRAS codons 61, 12, 13, etc. was about 15 to 20%, and when conducting the tests for RAS mutations other than KRAS exon 2, the RAS mutation rate was increased (Douillard et al. N Engl J Med. 2013).

In the treatment of cancers, it is very important to determine a proper drug as a primary anticancer chemotherapeutic agent. In case of colorectal cancers, prior to the development of biologic agents, the treatment of the cancers was conducted by the two methods (Toumigand, et al. JCO 2004): administering FOLFOX6 (fluoropyrimidine, leucovorin, oxaliplatin) or FOLFIRI as a primary therapeutic agent, and administering FOLFIRI or FOLFOX6 in reverse as a secondary therapeutic agent. However, as target therapeutic agents, bevacizumab and cetuximab, were developed, when adding bevacizumab to FOLFIRI in the primary treatment, the effect on prolonging the survival time was proven, and the CRYSTAL study showed the effect on prolonging the survival time when administering FOLFIRI in combination with cetuximab to a patient with a normal KRAS gene (Van Cutsem, J Clin Oncol. 2011).

In addition, along with the development of several target therapeutic agents, the studies on factors based on which the reaction to these agents could be predicted have been reported. The representative report is directed to the EGFR monoclonal antibody and mutations of KRAS gene, where since EGFR stimulates the growth and spread of cells through KRAS signaling pathway, if the KRAS signaling pathway is abnormal, that is, there are mutations of KRAS or BRAF, the effect of blocking EGFR may be reduced. Actually, KRAS mutations and BRAF mutations have been reported as indicators that can predict the reduction of the therapeutic effect of EGFR monoclonal antibody such as cetuximab or panitumumab (Eberhard DA, J Clin Oncol., 2005 / Karapetic CS, N Engl J Med, 2008/ Di Nicolantonio F, J Clin Oncol., 2008).

In this connection, in the small group analysis material on the gefitinib monotherapy phase 2 study material from Astrazeneca, the fact that the clinical characteristics of patients with radiological response to gefitinib are female, non-smoker, adenocarcinoma, and Asian was discovered, and this result was remarkably consistent with the result in the later erlotinib study material. In addition, even in the BR.21 study result, among the clinical factors associated with the erlotinib response, the significant differences of the surviving material in non-smoker, adenocarcinoma, and Asian could be overserved. Based on such clinical materials, a hypothesis that the small group of patients who respond to EGFR-TKI would have any molecular mechanism which can provide an explanation has been presented, and as a result of analysis of EGFR gene sequence based on the hypothesis of oncogene addiction, EGFR mutations have been discovered. In several tumors (breast cancer, glioma, prostate cancer, etc.), EGFR mutations have already been known, but it has been known that the positions of the occurrence thereof are almost extracellular domains and are not associated with EGFR-TKI response.

In contrast, as a result of confirming mutations that determine an EGFR-TKI response, it was confirmed that the mutations occurred within exon that determines a tyrosine kinase domain where EGFR-TKI functions in a cell, and also confirmed that those mutations are associated with an ATP-binding pocket or activation loop. EGFR tyrosine kinase domain is composed of 7 exons (18-24), and most of EGFR mutations are located at exons 18~21 and classified into the following three types: deletion of exon 19, occupying 60%; missense mutation (L858R) of exon 21, occupying 25%; and point mutation of exons 18, 20 and 21 and insertion/duplication of exon 20, occupying the other, which is rare. It was known that such EGFR mutations selectively activate Akt and STAT pathways that promote cell and antiapoptosis among downstream of EGFR signaling pathways, but do not affect ERK pathway associated with cell proliferation, so it was discovered that the use of EFGR-TKI induces apoptosis which leads to a prompt effect.

The mutations of EGFR tyrosine kinase domain were observed in about 20% of non-small cell lung cancers analyzed so far, and the mutations were observed in adenocarcinoma, Asian, female, and non-smoker patients, etc. with a remarkably high frequency; as such, the mutation rate exactly matches up with clinical indexes with a high EGFR-TKI response rate. However, it was reported that in case where there are EGFR mutations, the use of EGFR-TKI does not significantly increase the survival rate.

EGFR-TKI resistance - It has been known shortly after the introduction of EGFR-TKI in clinical trials that in almost of patients including patients who had dramatic responses to EGFR-TKI, a recurrence is finally developed. Recently, the fact that a secondary mutation (T790M) is generated at exon 20, so the resistance to gefitinib or erlotinib is acquired has been discovered, and it is assumed that this mutation is critical for disrupting a hydrogen bond of a drug in an ATP-binding pocket. This is another problem to be overcome in the use of EGFR-TKI, and the studies for developing EGFR-TKI which has no problem in acquired resistance are in progress.

As one of the factors that determine a response to EGFR-TKI, K-ras has recently been suggested, and it has been reported that patients with exon 2 mutation of K-ras rarely respond to EGFR-TKI. It was known that K-ras mutations are observed in 30% of adenocarcinoma; however, the fact that the mutations frequently occur in smoker and males, and the frequency of the occurrence thereof is low in Asia is almost exactly an opposite phenomenon to the EGFR mutations. Based on this, a hypothesis has been also proposed that in the carcinogenesis of adenocarcinoma, it would occur in non-smokers due to EGFR mutations; whereas it would occur in smokers due to K-ras mutations. The endogenous resistance problem other than the acquired resistance due to EGFR secondary mutation is also a task to be achieved for improving the response rate to EGFR-TKI; currently, it is assumed that as factors involved therewith, the role of EGFR ligand, the role of PTEN in the EGFR-non-dependent activation of PI3K/AKT and MAPK which are downstream of EGFR pathway, and oncogenic pathways other than EGFR (e.g., IGF-IR pathway), etc. may be associated.

Cetuximab (C225, Erbitux), which is a murine/human chimeric monoclonal antibody that binds to an extracellular water soluble portion, domain III of EGFR, prevents the dimerization of EGFR. It has been known that cetuximab exhibits the effect in the combination treatment with the existing cytotoxic anti-cancer therapy, as compared to its monotherapy. In the phase 1 study on the use of cetuximab in combination with cisplatin, it was confirmed that there is no cumulative toxicity, and in the phase 2 study conducted for patients with the expression of EGFR according to immunohistochemistry being over+1 among patients with recurrent non-small cell lung cancer, as a result of the combination treatment with docetaxel, it showed encouraging results that the response rate was 25% and the disease control rate was 60% or higher. Based on the results, the phase 3 study on the combination treatment with docetaxel or pemetrexed is currently in progress. The approval of cetuximab was currently obtained for colorectal cancers, and it has been reported that the treatment with cetuximab in combination with the radiotherapy exhibits the synergistic effect in locally advanced head and neck cancers.

As such, many target therapeutic agents, other than EGFR-TKI, gefitinib, erlotinib and bevacizumab currently approved, have been developed, and the active clinical studies have been conducted, and sensitive analysis of mutant types of a specific gene may provide a lot of information in the development of target therapeutic agents.

In particular, this may provide information as clues that solves problems, such as the fact that where gefitinib and erlotinib as monotherapeutic agents are currently effective only in limited patients in the secondary treatment, the acquired resistance problem, and the fact that the effect of bevacizumab as a primary therapeutic agent was proven, but this can be used only in some patients, etc. In addition, it can be variously utilized in studies of the treatment of a new target therapeutic agent in combination with a cytotoxic anti-cancer therapy, the combination therapy with targeted therapeutic agents, the role as post-operative adjuvant therapeutic agent, the role as a chemopreventive agent, the development of molecular biological markers on the selection of a target therapeutic agent, etc.

In addition, there is a report that an anti-EGFR antibody has an effect on inhibiting the proliferation of cancer cells by suppressing the RAS/MAPK mechanism that regulates the cell proliferation, but patients who have a KRAS mutation do not respond to the anti-EGFR antibody, and thus, when treating with a chemotherapy, it is very important to identify the genotype of a cancer patient (Herreros-Villanueva et al. Clin Chim Acta. 2014), and various testing methods and technologies for identifying the genotype of a cancer patient have been introduced.

Since the detection of mutations with clinical significance is very important, various detection methods for diagnosis according to the purpose of analysis or types of genes to be analyzed have been reported (Taylor CF, Taylor GR. Methods Mol. Med.; 92:9, 2004). However, since the number of mutant cancer cells present in a sample for analysis is significantly low as compared to normal cells, the detection thereof is very difficult, and a high level detection technique is required (Chung et al., Clin Endocrinol, 2006/ Trovisco et al, J Pathol., 2004).

Examples of gene mutation detection methods include Sanger sequencing, pyrosequencing, which are traditional methods, and real-time PCR, digital PCR, recently developed, etc. Sanger sequencing is capable of finding a new mutant form since it shows all sequencings, and reagents thereof are inexpensive.

Pyrosequencing has a high sensitivity, a fast speed and convenience in data analysis as compared to Sanger sequencing, but not all of mutant forms can be identified (Tan et al. World J Gastroenterol 2012). Real-time PCR can be conducted without special technical background, and can be performed rapidly with little manpower, and many advantages thereof, such as excellent sensitivity and reproducibility, and convenience in solving problems, have been reported.

In addition, as representative methods for detecting mutations contained in a small amount, the following various analysis methods based on the real-time PCR technique have been introduced: allele specific PCR method using a primer specific to a mutation in order to selectively increase the mutant gene products (Rhodes et al., Diagn molpathol., 6:49, 1997); scorpion Real-time allele specific PCR (DxS' scorpions and ARMS) method (Mark et al., Journal of Thoracic Oncology, 4:1466, 2009); CAST PCR method of inhibiting the amplification of a wild-type gene by using the allele specific primer technique and a MGB(minor groove binder)-probe and selectively amplifying only a mutant gene, and then detecting an amplification product without comprising a position where the mutation is generated by using a Taqman probe (Didelot A et al., Exp Mol Pathol., 92:275, 2012); a Cold-PCR method of increasing the sensitivity of mutation by using a critical denaturation temperature (Tc) (Zuo et al., Modern Pathol., 22:1023, 2009), etc. These methods provide mutation diagnosis and analysis of genes associated with various cancers easily and rapidly (Bernard et al., Clinical Chemistry, 48:1178, 2002).

PNA (Peptide nucleic acid) is a nucleic acid analogue having N-(2-aminoethyl) glycine amide as a backbone (Nielsen PE et al., Science, 254(5037):1497, 1991). The PNA backbone has a higher specificity than a DNA probe, with respect to a target nucleic acid having electrically neutralized complementary sequences. In addition, since the PNA has an advantage that this is not degraded by nuclease or protease, this is very useful in the molecular diagnosis method using a probe (Egholm et al., Nature, 365:556, 1993; Nielsen et al., Bioconjugate Chem., 5:3, 1994; Demidov, et al., Biochem. Pharmacol., 48:1310, 1994). By using the aforementioned advantage of PNA, PCR clamping technique was developed in 1993 (Henrik Orum et al., Nucleic Acids Res., 21:5332, 1993). This technique is a technology for inhibiting PCR amplification by combining a PNA probe with a gene that is not desired to be amplified. By using a PNA probe complementary to a wild-type gene, the amplification of the wild-type gene can be selectively inhibited in the PCR reactions, so that mutations present in a small amount can be detected rapidly and accurately as compared to wild-type genes.

In addition, various techniques using the PNA clamping technique have been reported. The PNA-LNA clamp method (US Patent Application Publication No. 2013-0005589; Yoshiaki Nagai et al. Cancer Res., 65(16):7276, 2005) is a selective amplification and detection method designed such that a PNA clamping probe which has the arrangement of a wild-type gene and a LNA taqman probe for detection which has the arrangement of a mutated gene competitively hybridize with respect to the target site. The PNA hyb probe (PNA as both PCR clamp and sensor probe; US Patent Application Publication No. 2008-0176226) is a method designed to perform clamping and detection simultaneously through a PNA probe by using the PNA probe, instead of a donor probe of the existing Roche's hyb probe system. The PNA clamping & intercalator detection method (Makito Miyake et al., Biochem Biophys Res Commun., 2007) is a method for clamping a wild-type gene by using a PNA probe and selectively amplifying only a mutant gene, and then detecting an amplification product by using an intercalator. As such, various techniques have been reported.

### DETAILED DESCRIPTION OF THE INVENTION

### TECHNICAL SUBJECT MATTER

Cancers arise due to mutation of genotype, and there are around 50-80 mutations that are absent in non-tumor cells ([Joneset al., 2008]; [Parsons et al., 2008]; [Wood et al., 2007]). Techniques for detecting these mutation profiles include the analysis of biopsy samples and the method for analysis of mutant tumor DNA circulating in body fluids such as blood (Diehl et al., 2008). However, the analysis of biopsy samples is invasive, and has a likelihood of maleficence such as complications. In addition, in the analysis method of biopsy samples, tissue samples are taken from a limited area and may give false positives or false negatives in tumors which are heterogeneous or dispersed within normal tissue. In order to overcome the problems of the methods, a non-intrusive and sensitive diagnostic method would be highly desirable.

In addition, tumor tissue may have heterogeneous types of mutations for each cell, which are mixed with normal cells; thus, for accurate mutation examination, the procedure for identifying tumor site through the use of a high-sensitive examination method and a microscopic examination, and selective DNA extraction and examination on the subject site are essential.

Further, in the analysis of mutant tumor DNA circulating in body fluids such as blood, the sensitivity is originally insufficient because the copy number of mutant cancer DNA is extremely low in the body fluids (Gormally et al., 2007). In order to overcome such disadvantage, the development of diagnosis methods for detecting tumor cells with high specificity and high sensitivity is required.

Several methods for detecting polymorphism of genes have been reported. The examples thereof include PCR (Polymerase Chain Reaction)-RFLP(Restriction Fragment Length Polymorphism) method, etc. The PCR-RFLP method is a method for, with respect to a target DNA of the sample, amplifying a region to be detected with PCR, treating the amplified product with restriction enzymes, and typing the change of the restriction fragment length due to polymorphism by Southern hybridization. If the mutation of interest exists in gene, the recognition site of the restriction enzymes disappears, so the presence of the mutation can be detected by the presence of cleavage, i.e., the change of the restriction fragment length. However, the PCR-RFLP method needs, for example, analysis after treating the amplified product obtained after PCR, with several restriction enzymes, and the process of treating the amplified product with restriction enzymes uses the amplified product performed primarily; thus, the result resulting therefrom may have an error.

Recently, a method for analyzing Tm(Melting Temperature) has been presented as a method for detecting polymorphism. The method for detecting polymorphism uses a probe complementary to a region containing a polymorphism to be detected to form a hybrid (double-stranded nucleic acid) between the nucleic acid and the complementary detection probe. Subsequently, this hybridization product is heat-treated, so that melting temperature of the hybrid being dissociated into a single-stranded nucleic acid due to the increase of temperature is detected by measuring the signal such as absorbance. The polymorphism is then determined based on the result of the Tm value.

The higher homology between each of single-stranded nucleic acids the hybrid has, the higher the Tm value is; and the lower homology it has, the lower the Tm value is. Therefore, the Tm value (reference value for assessment) is determined for the hybrid between the gene and the complementary probe and then the reference value is compared with the Tm value (measured value) between the nucleic acid to be examined and the complementary probe to determine whether the site to be detected in examined nucleic acid is identical to the gene of interest. In addition, if the measured value is lower than the reference value for assessment, it can be determined that the site to be detected in the examined nucleic acid has a different profile.

According to such method, the polymorphism can be detected only by performing the signal measurement after PCT reaction in which a detection probe is added. For such a detection method using Tm analysis, the difference of 1 base should be determined with Tm value, and in case where a gene has a plurality of polymorphisms, the result analysis should be easy. In particular, even in case where a wild-type polymorphism and a plurality of mutant polymorphisms exist together, it is required to detect the presence of the mutation accurately. According to the present invention, a probe can be designed to allow the detection of the difference of 1 base through Tm analysis.

The detection of polymorphisms of a gene is important in, for example, diagnosis of a disease and selection of a treatment method. Therefore, the object of the present invention is to provide a probe for detecting polymorphisms, which is capable of determining polymorphisms of disease-related genes, EGFR, KRAS, NRAS, with simple and excellent sensitivity, and the use thereof.

Examples of gene mutation detection methods include Sanger sequencing, pyrosequencing, which are traditional methods, and real-time PCR, digital PCR, recently developed, etc. Sanger sequencing is capable of finding a new mutant form since it shows all sequencings, and reagents thereof are inexpensive. However, Sanger sequencing has disadvantages of low sensitivity, long examination period, and being capable of subjective analysis. Pyrosequencing, which is an intermediate stage of Sanger sequencing and real-time PCR, has a high sensitivity, a fast speed and convenience in data analysis as compared to Sanger sequencing, but by this sequencing, not all of mutant forms can be identified. In addition, when compared with Real-time PCR, by pyrosequencing, a specific mutant form can be found and new gene mutation can be identified through data analysis as compared to Real-time PCR, but the examination time is longer and the sensitivity is lower (Tan et al. World J Gastroenterol 2012 October 7; 18(37):5171-5190).

In particular, real-time PCR applied in this technology can be conducted without special technical background, and can be performed rapidly with little manpower. In addition, sensitivity and reproducibility are excellent, and when a problem occurs, it is easy to solve the problem. However, since a gene is found for the changed sequencing, all mutant forms cannot found.

Therefore, the present invention provides a method for detecting an extremely small amount of mutations with high detection sensitivity, and is to use the method for monitoring of the entire cycle of a related disease, disease prognosis and prediction, decision of disease treatment strategy, disease diagnosis/early diagnosis, disease prevention, and development of disease therapeutics.

### MEANS FOR SOLVING THE SUBJECT MATTER

In order to detect a target nucleic acid real-time more sensitively, it was found that, by using a mixture of a clamping probe and a fluorescent detection probe comprising a fluorescent substance and a quencher simultaneously, it is possible to selectively detect the desired genes with high sensitivity, and it was found that it is possible to easily detect adjacent mutations.

Also, it was found that by confirming the difference in the melting temperature between the wild-type gene and target nucleic acid gene, it is possible to detect multiple target nucleic acids simultaneously and determine the genotypes thereof through melting curve analysis.

In order to detect a target nucleic acid real-time more sensitively, the present invention provides a probe mixture for real-time detection of target nucleic acids, comprising at least one detection probe and at least one clamping probe for inhibiting amplification of wild type genes or unwanted genes, a method for simultaneous detection of multiple target nucleic acids using the probe mixture, and a kit for diagnosing molecules using the method.

The probe mixture of the present invention provides a method for simultaneous detection of multiple target nucleic acids using a clamping probe and at least one detection probe to which a reporter and a quencher are attached, both of which are competitively hybridized with the same strand, and the use thereof.

The probe mixture system of the present invention can be used in various molecular diagnosis technologies such as molecular diagnosis, prenatal diagnosis, early diagnosis, cancer diagnosis, genetic associated diagnosis, diagnosis of genetic character, diagnosis of bacterial infection, determination of drug resistant bacterium, forensic medicine, determination of species of living organism, etc.

In particular, in order to provide a cancer patient with proper diagnosis and treatment, the identification of various molecular properties of cancer types is required. In addition, according to the technology of the present invention with more sensitivity and higher specificity than the basic method, the detection of the expression of a small amount of gene, point mutations, a fusion gene, etc. contained in a normal DNA is possible, which makes it possible to understand the entire genetic basis of cancer and apply for the diagnosis and treatment.

In general, the present invention supports early diagnosis/diagnosis, monitoring and evaluation of a related disease, another medical condition, and treatment efficacy, by sensitively detecting mutant forms such as EGFR, KRAS, NRAS, etc. contained in body fluids, especially, blood. In addition, the present invention may comprise the steps of comparing the result of the detection of a mutation related to a disease with a normal control group to identify the association between the mutant form and the disease or other medical condition (e.g., cancer). Further, the present invention makes it possible to determine the treatment method and the drug reactivity prediction through the genotype identification.

### EFFECT OF THE INVENTION

By using the method for detecting target nucleic acids according to the present invention, it is possible to inhibit amplification of wild-type genes or unwanted genes. Also, through selective amplification and selective detection of a very small amount of target nucleic acid genes, the method allows to effectively detect single nucleotide variation and mutation caused by loss or insertion of base in a sample. Also, by using multiple detection probes and multiple amplification inhibition probes, the method enables to simultaneously analyze real-time amplification curve and melting curve, which allows to not only simultaneously detect and quantify multiple target nucleic acids but also determine genotype by melting curve analysis. Also, the method makes it possible to detect the target with high sensitivity, and thus can be very useful for early diagnosis requiring the detection of a trace of the target.

By the probe for detecting polymorphism according to the present invention, a small amount of mutations contained in, for example, EGFR, KRAS, NRAS genes can be determined by Tm analysis simply and with excellent reliability. More specifically, even in case where genes of interest in a wild-type and a mutant-type co-exist in a sample, by performing Tm analysis using the probe for detecting polymorphism according to the present invention, the types of polymorphism or the presence of mutation can be detected simply and with excellent reliability. For this reason, the present invention is useful especially for a sample in which a large amount of wild-type genes and a small amount of mutant genes are contained. According to the present invention, the polymorphism of EGFR, KRAS, NRAS genes can be determined simply and with excellent reliability; thus, for example, the detected result can be reflected in diagnosis of a disease and selection of a treatment method, etc. aforementioned. Therefore, the present invention is very useful in medical field, etc.

### BEST MODE FOR CARRYING OUT THE INVENTION

The present invention relates to a method for simultaneous detection of multiple target nucleic acids using a clamping probe for inhibiting amplification of wild-type genes or unwanted genes and a detection probe to which a reporter and a quencher are attached, and the use of the detection method.

The detection probe in the present invention means a probe that can selectively detect a target nucleic acid gene to be detected. The clamping probe means a probe that can inhibit the elongation reaction by polymerase between PCR reactions by complementarily binding to wild-type genes or unwanted genes, and the probe mixture means a probe system comprising at least one detection probe and at least one clamping probe.

The target nucleic acid in the present invention means all types of nucleic acids to be detected, and may include or may not include a mutant gene. This can be characterized by all types of DNAs including genomic DNA, mitochondrial DNA, and viral DNA or all types of RNAs including mRNA, ribosomal RNA, non-coding RNA, tRNA, viral RNA, etc., but is not limited thereto. It is annealed or hybridized with a primer or a probe under hybridizing, annealing or amplifying conditions.

The hybridization in the present invention means complementary single strand nucleic acids forming a double-strand nucleic acid. Hybridization occurs when two nucleic acid base sequences are in a perfect match or may occur even when some mismatch bases exist. The degree of complementarity required for hybridization may vary depending on hybridization conditions such as temperature.

The mutation in the present invention means a variation in the base sequence of wild-type gene, including not only single nucleotide polymorphism (SNP) but also variation caused by substitution, loss or insertion of base. Also, mutation includes somatic mutation and germline mutation that may occur naturally, and also includes, without limitation, artificial mutation, etc. where the variation in the sequence was artificially induced.

The somatic mutation in the present invention is known as the main cause of tumorigenesis by deregulation in signal transduction process, etc. Examples of significant somatic mutation include various cancer related genes such as KRAS, BRAF, EGFR, JAK2, HER2, BCL-ABL, NRAS, HRAS, IDH1, IDH2, C-KIT, TP53, EGFR, PIK3CA, etc. In the present invention, it was confirmed that for mutations of the somatic genes EGFR, KRAS, NRAS, the present invention works, and the application field was presented.

In the present invention, the detection probe or clamping probe can be any nucleic acid or nucleic acid analogue complementarily binding to the target nucleic acid, selected from the group consisting of oligonucleotides, PNA (peptide nucleic acids) and LNA (locked nucleic acid). In general, polymerase for PCR has nuclease activity, which may lead to a damage to a probe. Thus, it is preferable to use synthetic nucleic acid such as PNA, which is stable to nuclease. Also, since the detection probe plays a role of selectively detecting the target nucleic acid gene, probes for the detection of nucleic acids which are well known in the pertinent art can be used.

In one embodiment of the present invention, preferably, a PNA probe is used. The PNA probe is an artificially synthesized DNA and can specifically bind to the target DNA or RNA. Since PNA probe is stable against nuclease, it allows a probe-based melting curve analysis. Also, PNA probe has a property of inhibiting the progress of polymerase after binding to DNA. Thus, in the present invention, the PNA probe designed to be completely hybridized with the wild-type gene is used as a PNA clamping probe, and the PNA detection probe for simultaneous detection of multiple target nucleic acids is designed to be completely hybridized with the target nucleic acid gene.

Also, in accordance with the present invention, modification can be made to the three-dimensional structure of the clamping probe and detection probe by attaching a specific group such as the side chain of a natural amino acid or synthetic amino acid at the N-terminus, C terminus of the probe or the alpha, beta, gamma, linker position of the probe (synthetic nucleic acid) backbone. The amino acid may or may not have an electric charge, or may have a negative or positive charge, but is not limited thereto. Any method for changing the three-dimensional structure of probe or imparting electric charge which are known in the pertinent art can be used.

The detection probe in the present invention can have a reporter and a quencher capable of quenching reporter fluorescence attached at both terminals thereof, and can include an intercalating fluorescent substance. The reporter refers to a substance absorbing and emitting light of a specific wavelength to emit fluorescence, and which labels a probe to identify whether the target nucleic acid and the probe were hybridized. The reporter can be at least one selected from the group consisting of fluorescein, fluorescein chlorotriazinyl, rhodamine green, rhodamine red, tetramethylrhodamine, FITC, oregon green, Alexa Fluor, FAM, JOE, ROX, HEX, Texas Red, TET, TRITC, TAMRA, cyanine-based dye and thiadicarbocyanine dye, but it not limited thereto. Any substances emitting fluorescence which are well known in the pertinent art can be used.

Also, the quencher means a substance absorbing the light generated by the reporter to reduce the strength of the fluorescence. The quencher can be at least one selected from a group consisting of Dabcyl, TAMRA, Eclipse, DDQ, QSY, Blackberry Quencher, Black Hole Quencher, Qxl, Iowa black FQ, Iowa black RQ, IRDye QC-1, but is not limited thereto. Quencher reduces the strength of fluorescence to a different extent depending on its type, and thus may be used in consideration of this matter.

The method for simultaneous detection of multiple target nucleic acids using the probe mixture of the present invention is characterized by using one or at least one clamping probe inhibiting the elongation of polymerase by complementarily binding to wild type genes or unwanted genes to selectively amplify the target nucleic acid gene to be detected, and using one or at least one detection probe for specifically detecting the target nucleic acid to detect the presence or concentration of multiple target nucleic acids. Detection of the target nucleic acid using a probe mixture allows the simultaneous analysis of real-time amplification curve and melting curve.

The clamping probe and detection probe of the present invention hybridize with the same strand of the target DNA, or the clamping probe and detection probe hybridize with complementary strands, thereby blocking wild type genes and detecting target nucleic acid genes simultaneously. That is, if the clamping probe is hybridized with a perfect match with the wild-type gene, amplification of the wild-type gene can be inhibited, which makes it possible to selectively amplify and detect a trace of the target nucleic acid gene. Also, it is possible to simultaneously detect multiple target nucleic acids, by using at least one detection probe.

In the process of amplifying the target DNA using a probe mixture, in the annealing step, each of the clamping probe and detection probe is annealed to the same strand or different complementary strands. The detection probe having a reporter and a quencher specifically binds to the target nucleic acid gene to be detected, thereby emitting an amplification curve signal (fluorescence). In the subsequent extension step, the clamping probe is still hybridized with the wild-type gene or unwanted genes, and thus inhibits amplification of the wild-type gene or unwanted genes. The detection probe is separated from the target nucleic acid to allow amplification to proceed, because it is designed to have a melting temperature lower than the temperature of the extension of the target nucleic acid.

The amplification process allows a real-time analysis of the amplification curve. Also, a melting curve analysis is possible by using the amplification product generated by the amplification process. In the melting curve analysis step, at low temperature, the detection probe is hybridized with the target nucleic acid gene, thereby emitting a fluorescence signal, but as the temperature rises, it is separated from the target nucleic acid and thus fluorescence is quenched.

In general, in order to detect a plurality of target nucleic acids simultaneously, the conventional methods for analyzing amplification curve using real-time PCR use a fluorescent substance to detect the target nucleic acid in a sample. Thus, the methods have a problem that as many probes having a fluorescent substance as the number of targets is required in order to detect at least two target nucleic acids, and thus have a limitation in multiple detection. However, the method for simultaneous detection of multiple target nucleic acids of the present invention can conduct an amplification curve analysis and a melting curve analysis simultaneously by using a probe mixture, and thus is capable of detecting multiple targets by using one fluorescent substance.

In one embodiment of the present invention, in order to confirm the difference in melting temperature, PNA probes can be synthesized by introducing the side chain of negatively charged L-glutamic acid or D-glutamic acid, uncharged L-alanine or D-alanine, or of positively charged L-lysine or D-lysine at the gamma position of PNA, and by introducing L-lysine or L-glutamic acid at the linker position.

When compared with the PNA probe with no modified structure, the PNA probes structurally modified (in terms of three-dimensional structure and electric charge) by attaching D-glutamic acid at the gamma position of the PNA backbone showed a great increase in the difference (ΔTm) of the melting temperature between the target DNA and single nucleotide mismatch DNA. Also, it was confirmed that the PNA probe of the present invention with a modified structure has an increased specificity to single nucleotide variation, thereby achieving a remarkable difference in melting temperature between the wild-type gene and target nucleic acid gene, which allows to reduce non-specific binding in real-time amplification curve and problems that may occur in melting curve analysis.

In general, in order to detect a plurality of target nucleic acids simultaneously, the conventional methods for analyzing amplification curve using real-time PCR use a fluorescent substance to detect the target nucleic acid in a sample. Thus, the methods have a problem that as many probes having a fluorescent substance as the number of targets is required in order to detect at least two target nucleic acids, and thus have a limitation in multiple detection. However, the method for simultaneous detection of multiple target nucleic acids of the present invention can conduct an amplification curve analysis and a melting curve analysis simultaneously by using a probe mixture, and thus is capable of detecting multiple targets by using one fluorescent substance.

Also, the method for simultaneous detection of multiple target nucleic acids using the probe mixture of the present invention is not limited to simultaneous analysis of amplification curve and melting curve. It also enables a separate or sequential analysis of amplification curve and melting curve. The method also allows to detect target nucleic acids by performing only amplification curve analysis or melting curve analysis, as needed. Particularly, in case where quantitative analysis is not required, by conducting only a curve analysis alone without an amplification curve analysis, the presence or genotype of the target nucleic acid can be determined.

Specifically, the method for simultaneous detection of multiple target nucleic acids of the present invention comprises: (a) performing hybridization by mixing a primer and a probe mixture composed of a clamping probe and a detection probe in a test specimen including the target nucleic acids, and obtaining a real-time amplification curve; (b) obtaining a melting curve between an amplified product and the detection probe with changing temperature after the amplification; and analyzing the obtained real-time amplification curve and melting curve separately, sequentially or simultaneously.

The clamping probe and detection probe in step (a) can be variously adjusted according to the number of target nucleic acids to be detected. In order to decrease the deviation of melting temperature depending on the concentration of the amplification product in the step of obtaining the melting curve, before the step of obtaining the melting curve, at least 5 PCR cycles can be added besides the step of obtaining real-time amplification curve. Preferably, 5~20 cycles can be added.

Also, a detection method of the present invention may use the method for simultaneous detection of multiple target nucleic acids comprising: (a) performing hybridization by mixing a primer and a probe mixture composed of a clamping probe and a detection probe in a test specimen including the target nucleic acids, and obtaining a real-time amplification curve; and (b) analyzing the obtained real-time amplification curve, or

the method for simultaneous detection of multiple target nucleic acids comprising:
(a) performing hybridization by mixing a primer and a probe mixture composed of a clamping probe and a detection probe in a test specimen including the target nucleic acids;
(b) obtaining a melting curve by melting the hybridized product with changing temperature; and
(c) analyzing the obtained dissociation curve can be used.

The step of obtaining an amplification curve or melting curve in the present invention is performed through the real-time PCR (polymerase chain reaction), and the amplification curve analysis is characterized by measuring and analyzing the Ct (cycle threshold). If the target nucleic acid exists in the sample or a large amount of the target nucleic acid is included in the sample, the number of cycles required to reach the threshold decreases, thus resulting in a low Ct value. Thus, this analysis enables to confirm the presence of the target nucleic acid and to detect the amount of the initial target nucleic acid.

Also, in general, the melting curve analysis is performed lastly after the process of real-time PCR is completed. In this analysis, after lowering the temperature of the sample to around 30~55°C, intensity of fluorescence signal is measured while increasing the temperature by 0.5~1°C every second up to 95°C. When the temperature goes up, the detection probe and the target nucleic acid (one strand of the target nucleic acid that can complimentarily bind to the detection probe) are separated from each other, and then fluorescence is quenched, which results in a sharp decline in fluorescence signal. Accordingly, it is possible to confirm the presence of a target nucleic acid through the melting peak.

The method for simultaneous detection of multiple target nucleic acids of the present invention is characterized by detecting target nucleic acid included in an amount of 0.01% or 0.1%~100% in 10 ng or below of a nucleic acid sample.

The term "sample" in the present invention covers various samples. Preferably, biosamples are analyzed using the method of the present invention. Biosamples of the origin of plants, animals, human beings, fungus, bacteria and virus can be analyzed. In case of analyzing samples of the origin of mammals or human beings, the sample can be originated from a specific tissue or organ. Representative examples of the tissues include connective tissues, skin tissues, muscle tissues or nervous tissues. Representative examples of the organs include eye, brain, lung, liver, spleen, bone marrow, thymus, heart, lymph, blood, bone, cartilage, pancreas, kidney, gallbladder, stomach, small intestine, testis, ovary, uterus, rectum, nervous system, gland and internal vessel. The biosample to be analyzed includes any cell, tissue, or fluid of the biological origin or any medium that can be well analyzed by the present invention, which include samples obtained from foods produced to be consumed by human beings, animals or human beings and animals.

Also, the biosample to be analyzed includes body fluid samples, which include blood, blood serum, plasma, lymph, breast milk, urine, human feces, eyeball fluid, saliva, semen, brain extract (for example, brain splinters), spinal fluid, and extracts from appendix, spleen, and tonsil tissues, but are not limited thereto.

The target nucleic acid of the sample is DNA or RNA, and the molecule may be in the form of a double strand or a single strand. In case the nucleic acid as an initial substance is double stranded, it is preferable to make the double strand into a single strand, or a partially single-stranded form. Well known methods for separating strands include heat treatment, alkali treatment, formamide treatment, urea treatment and glycoxal treatment, enzymatic methods (e.g., helicase action) and binding protein, but are not limited thereto.

According to another aspect, the present invention relates to a kit for simultaneous detection of multiple target nucleic acids comprising a probe mixture composed of a clamping probe and a detection probe, the kit using the method for simultaneous detection of multiple target nucleic acids of the present invention, and the use thereof.

According to another aspect, the detection method of the present invention can be used for diagnosis, prognosis and monitoring of the medical condition of a disease, treatment efficacy evaluation, and for supporting nucleic acid and protein delivery studies and so on, through a very small amount of a mutant genotype that is confirmed at a high detection sensitivity.

The present invention provides a method comprising a step for detecting biomarkers such as EGFR, KRAS, NRAS etc. and evaluating the presence of mutations of the biomarkers by using not only invasive specimens such as tissues but also non-invasive specimens (blood, urine, sputum, stool, saliva, and cells), wherein the presence of the biomarker and mutations is used for monitoring of the entire cycle of a related disease, disease prognosis and prediction, decision of disease treatment strategy, disease diagnosis/early diagnosis, disease prevention, and development of disease thereapeutic agents.

In this regard, the present invention has an advantage of performing various gene tests at once more rapidly and inexpensively. Particularly, if only the modification of genes determining a therapeutic agent of a specific cancer is detected by applying the present technology, it is possible to find out modifications of all genes related to the related disease with current costs for testing one gene. In this regard, for lung cancer patients, it should be examined whether EML4-ALK, ROS1, KIF5B-RET genes are fused, together with the test for mutations of genes such as EGFR, KRAS, HER2, MET, TP53, NRAS, PIK3CA, etc., and for colorectal cancer patients, tests on all mutations of genes such as KRAS, BRAF, TP53, NRAS, PIK3CA, etc. should be conducted, so that the tests may give clinical helps to these patients.

In addition, the present invention is a technology related to clinical genomics, which allows personalized medical treatment rapidly based on personal gene information and may be variously utilized in very many fields for medical expenses, loss due to side effects of a drug, reducing costs for developing new drugs, etc. Particularly, the present invention will give an opportunity to select more proper therapeutic agents based on the progress of various clinical studies on pharmacotherapies according to a specific genotype, for Asian.

Genetic diseases are diseases caused by genetic abnormalities, and the genetic abnormalities present in somatic cells display phenotypes. Since the same genetic abnormality is also present in germ cells (sperm, ovum), the phenotypes refer to all diseases delivered to descendants. Causes resulting in genetic abnormalities are firstly, an error naturally occurring when DNA is replicated, and secondly, the possibility that the genetic abnormalities may occur due to several chemical substances and environmental factors. According to the human genome project research results recently reported, 30,000 - 40,000 genes exist in the cells of human, and according to the results, it may be assumed that there are numerous types of human genetic diseases. It can be predicted that there are many types of genetic diseases, other than the types of genetic diseases discovered so far, and there are much more genetic diseases that have not been studied until now.

Study on genes through excellent detection sensitivity in the present invention is expected to be utilized as a means for predicting the types of genetic diseases that have not been discovered. For example, in K-ras gene, if at least one of polymorphisms of codon 12 and polymorphisms of codon 13 carries a mutation, it could be determined that it exhibits resistance to anti EGFR antibody drug such as cetuximab, etc. and if it is wild-type, it does not exhibit resistance.

The occurrence of multifactorial genetic diseases greatly depends on the environment, and the heredofamilial possibility also varies depending on entities and related diseases, which is referred by genetic polymorphism. Genetic polymorphism means that the same gene for each person is expressed differently, and the examples of the types include STR(short tandem repeat), SNP(single nucleotide polymorphism), etc. STR means repeatedly displaying 2 to 5 short base sequences, and SNP means about one base sequence being different per 1.0kb in the gene. STR is used for parentage test, and SNP is widely used and studied for the likelihood or prognosis determination of a disease and a response to a specific drug as well as personal identification.

One present in an amount of 1% in the group of genetic polymorphism is called "mutation." Recently, studies on genetic polymorphism and mutations in main diseases such as cancer, diabetes, Alzheimer's disease, etc. have been actively conducted, and for such studies, the application of the present technology is possible.

In order to proceed with the detection of mutations of a gene, a method of extracting DNA of a cancer cell contained in the sample and analyzing the gene of a patient through various technologies is used. When detecting a mutation of gene, the sensitivity of the detection method is important. Sensitivity means how much % of mutation can be detected in the entire gene, and it is reported that Sanger sequencing has a false negative rate of 10%, pyrosequencing has a false negative rate of about 5%, and new methods recently developed have false negative rate of 1% or 0.1%, which are excellent in sensitivity (Nam et al. Korean J Pathol. 2014). In addition, it has been reported that since the gene analysis result varies depending on the quality of the sample, for accurate gene detection, tighter management of the molecular detection process is important (19. Nam et al. Korean J Pathol. 2014).

Diagnostic test of gene is conducted through several tests for the purpose of confirming or identifying a genetic disease suspected in a patient having the symptom of the disease. In addition, it is also possible to predict a disorder with a higher likelihood and risk of a disease by conducting a test for the presence of mutation of a gene which causes a disorder in a person who has a family history of genetic diseases. Such genetic tests are helpful in the prevention, early diagnosis, treatment and management of a disease, which results in reducing the morbidity rate and the mortality of the disorder.

Particularly, genetic tests can be conducted with any tissues of a human body, based on the premise that all cells in the human body are composed genetically in the same manner, and more easily, the genetic tests can be conducted by using DNA extracted from the blood. In addition, in order to detect variations related to genetic diseases in a gene, examples of samples include DNA, RNA, chromosomes, metabolites, and the like. The examples of the methods for the detection of gene include a method of directly analyzing DNA and RNA, and biochemical test or cytogenetic test for indirectly confirming the inheritable genotype together with a disease gene and analyzing metabolites.

Genotype examination can be conducted by various methods depending on the purpose of the examination. In particular, genetic SNPs and mutations can be detected by hybridization with allele-specific probes, enzymatic mutation detection, chemical cleavage of mismatched heteroduplex (Reference [Cotton et al., 1988]), ribonuclease cleavage of mismatched bases (Reference [Myers et al., 1985]), mass spectrometry (US Patent Nos. 6,994,960, 7,074,563, and 7,198,893), nucleic acid sequencing, single strand conformation polymorphism (SSCP) (Reference [Orita et al., 1989]), denaturing gradient gel electrophoresis (DGGE) (Reference [Fischer and Lerman, 1979a]; [Fischer and Lerman, 1979b]), temperature gradient gel electrophoresis (TGGE) (References [Fischer and Lerman, 1979a]; [Fischer and Lerman, 1979b]), restriction fragment length polymorphisms (RFLP) (References [Kanana Dozy, 1978a]; [Kan and Dozy, 1978b]), oligonucleotide ligation assay (OLA), allele-specific PCR (ASPCR) (US Patent No. 5,639,611), ligation chain reaction (LCR) and its variants (References [Abravaya et al., 1995]; [Landegren et al., 1988]; [Nakazawa et al., 1994]), flow-cytometric heteroduplex analysis (WO/2006/113590) and combinations/modifications thereof. Notably, gene expression levels may be determined by the serial analysis of gene expression (SAGE) technique (Reference [Velculescu et al., 1995]). The appropriate method of analysis will depend upon the specific goals of the analysis, the condition/history of the patient, and the specific cancer(s), diseases or other medical conditions to be detected, monitored or treated.

Aspects of the present invention relate to a method for monitoring disease (e.g. cancer) progression in a subject, and also to a method for monitoring disease recurrence in an individual. Aspects of the present invention also relate to the fact that a variety of non-cancer diseases and/or medical conditions also have genetic links and/or causes, and such diseases and/or medical conditions can likewise be diagnosed and/or monitored by the methods described herein.

Diseases or other medical conditions for which the inventions described herein are applicable include, but are not limited to, nephropathy, diabetes insipidus, diabetes type I, diabetes II, renal disease glomerulonephritis, bacterial or viral glomerulonephritides, IgA nephropathy, Henoch-Schonlein Purpura, membranoproliferative glomerulonephritis, membranous nephropathy, Sjogren's syndrome, nephrotic syndrome minimal change disease, focal glomerulosclerosis and related disorders, acute renal failure, acute tubulointerstitial nephritis, pyelonephritis, GU tract inflammatory disease, Pre-clampsia, renal graft rejection, leprosy, reflux nephropathy, nephrolithiasis, genetic renal disease, medullary cystic, medullar sponge, polycystic kidney disease, autosomal dominant polycystic kidney disease, autosomal recessive polycystic kidney disease, tuberous sclerosis, von Hippel-Lindau disease, familial thin-glomerular basement membrane disease, collagen III glomerulopathy, fibronectin glomerulopathy, Alport's syndrome, Fabry's disease, Nail-Patella Syndrome, congenital urologic anomalies, monoclonal gammopathies, multiple myeloma, amyloidosis and related disorders, febrile illness, familial Mediterranean fever, HIV infection-AIDS, inflammatory disease, systemic vasculitides, polyarteritis nodosa, Wegener's granulomatosis, polyarteritis, necrotizing and crecentic glomerulonephritis, polymyositis-dermatomyositis, pancreatitis, rheumatoid arthritis, systemic lupus erythematosus, gout, blood disorders, sickle cell disease, thrombotic thrombocytopenia purpura, Fanconi's syndrome, transplantation, acute kidney injury, irritable bowel syndrome, hemolytic-uremic syndrome, acute corticol necrosis, renal thromboembolism, trauma and surgery, extensive injury, burns, abdominal and vascular surgery, induction of anesthesia, side effect of use of drugs or drug abuse, circulatory disease myocardial infarction, cardiac failure, peripheral vascular disease, hypertension, coronary heart disease, non-atherosclerotic cardiovascular disease, atherosclerotic cardiovascular disease, skin disease, soriasis, systemic sclerosis, respiratory disease, COPD, obstructive sleep apnoea, hypoia at high altitude or erdocrine disease, acromegaly, diabetes mellitus, or diabetes insipidus. The cancer monitored or otherwise profiled, can be any kind of cancer. This includes, without limitation, epithelial cell cancers such as lung, ovarian, cervical, endometrial, breast, brain, colon and prostate cancers. Also included are gastrointestinal cancer, head and neck cancer, non- small cell lung cancer, cancer of the nervous system, kidney cancer, retina cancer, skin cancer, liver cancer, pancreatic cancer, genital-urinary cancer and bladder cancer, melanoma, and leukemia. In addition, the methods and compositions of the present invention are equally applicable to detection, early diagnosis/diagnosis and prognosis of non-malignant tumors in an individual (e.g. neurofibromas, meningiomas and schwannomas).

Differences in their nucleic acid content can be identified by analyzing the nucleic acid obtained from a body fluid of one or more subjects with a given disease/medical condition (e.g. a clinical type or subtype of cancer) and comparing to the nucleic acid of one or more subjects without the given disease/medical condition. The differences may be any genetic aberrations including, without limitation, expression level of the nucleic acid, alternative splice variants, chromosome number variation (CNV), modifications of the nucleic acid, single nucleotide polymorphisms (SNPs), and mutations (insertions, deletions or single nucleotide changes) of the nucleic acid. Once a difference in a genetic parameter of a particular nucleic acid is identified for a certain disease, further studies involving a clinically and statistically significant number of subjects may be carried out to establish the correlation between the genetic aberration of the particular nucleic acid and the disease.

The development of various technologies is needed for the medical development for personalized prevention, diagnosis, treatment by taking into consideration an individual gene, protein and environmental information. For example, there is a report that currently, for non-small cell lung cancer patients, a therapeutic agent is selected by identifying EGFR mutation or a single gene mutation with a method such as Sanger DNA base sequence analysis or RT-PCR, FISH, etc., but in near future, the whole genome or exome, and transcriptome of a patient is tested with the present technology, which will be helpful in personalized treatment.

If the disease is caused by a mutation in the gene, it could be treated if the gene compromised is altered. For example, in the United States in 1990, the T-lymphocyte was extracted from a girl with a severe congenital immunodeficiency due to a deficiency of a gene making an enzyme called adenosine deaminase (ADA), and then the ADA gene was put into this lymphocyte and then injected into the blood of the patient, and as a result, the immune function could be restored. As such, treating a disease with a gene is called gene therapy.

Gene therapy can be classified into *ex-vivo* gene therapy for extracting a target cell, inserting a specific gene, and then injecting it again as in the previous example, and *in-vivo* gene therapy for directly injecting a specific gene into a target tissue to obtain a therapeutic effect. Most of the recent gene therapies are tried for the purpose of treating mainly cancers. The therapies can be divided into a method of directly injecting an anti-gene that directly kills cancer cells into the cancer cells or a gene that makes the cancer cells sensitive to an anticancer agent, and a method of injecting genes that secrete various cytokines into white blood cells to infiltrate cancer cells or secrete cytokines themselves, which triggers immune response. Cancer, acquired immune deficiency syndrome (AIDS), and single gene disorders, etc. are currently the primary goals of gene therapy. It is possible to provide various information to the genetic confirmation method for gene therapy. In addition, studies on differences from genetic polymorphisms in specific populations or ethnic groups have been actively conducted, and for these studies, the present technology can be utilized.

In addition, in case of cancers, it is very difficult to know whether the gene makes certain carcinogens more weakened, or makes the function of restoring the destroyed gene decreased, or makes the immune function involved in cancer prevention weakened, or enables all of them complexly. Therefore, one gene may be involved in several diseases at the same time, and several genes may be related to one disease. Also, in case of multifactorial genetic diseases, even if the gene is present, the disease does not necessarily occur. For example, the most well-known cancer gene is a mutated BRCA1 gene, which is associated with breast cancer. Up to 65 years of age, the breast cancer may occur in 60% of women with this mutation. On the other hand, about 12% of normal females suffer from this cancer. This is a very common example of western breast cancer, and Korea is not necessarily because of environmental differences such as dietary life. However, this gene accounts for only 3% of all breast cancers, and there are so many other unknown factors. And, the fact that 60% of the persons with this mutation suffer from breast cancer means that all of the persons with this gene does not necessarily suffer from this disease. However, it is sure that this gene has great significance in the diagnosis and treatment of breast cancer. There are not so many genes with such high correlation in other diseases, and the techniques and methods of the present invention with higher sensitivity are likely to enable the discovery of highly correlated genes. In addition, the studies on the difference of drug response according to genetic polymorphism can provide various information for personalized medical treatment.

Cancer is a genetic disease, and cancer cells are formed by various gene mutations while environmental stimuli is added to the genetic tendency, and the cells do propagate and mutate. Recent important concepts are oncogenic addiction and synthetic lethality. Oncogenic addiction is a theory that the continuous activation of abnormal signaling pathways due to one or two gene abnormalities among various genetic abnormalities plays a key role in the development, growth and maintenance of cancer, which makes the gene inactivated, and thus the cancer can be treated.

Synthetic lethality is a concept that when suppressing each gene, cancer cells cannot be killed, but when suppressing two genes simultaneously, the cancer cells can be destroyed. As such, by finding the genes that role a main key and understanding the interaction of the genes, better therapeutic effects can be achieved, and by using the present technology, various information can be utilized.

In particular, unlike samples of other diseases, samples of cancer have various information on genomic DNA and somatic cell cancer genome of various sites such as primary cancer sites and metastatic cancer sites, and thus the samples can be utilized for various clinical trials. Thus, the present invention can be utilized for the evaluation of cancer risk and the early diagnosis of individual cancer through the analysis of single nucleotide polymorphism (SNP) and chromosome number variation (CNV) of the sample genotype.

Examples of cancer-specific somatic cell DNA variations include mutations, amplifications, deletions, fusions, and methylations of specific genes. The results from analyzing them are used not only for the assessment of cancer onset risk but also for diagnosis and treatment of individual patients. In addition to DNA variations, the degree of expression of RNA and protein, the degree of immune response of an individual, or the degree of secretion of cytokines may be used in the assessment of cancer onset risk and in the diagnosis and treatment of individual patients.

Currently, the most common cancer genomic information is the mutation of oncogenes detected in each cancer tissue or blood. The key mutation (driver mutation), most of which occurs in oncogenes, is the major genetic variation that determines the response of the patient to cancer development, progression, and therapeutic agent.

As the genetic variation is detected more sensitively, it can be used as a tool for finding the appropriate treatment method by monitoring the transgene variation in each cancer tissue or blood for personalized medical treatment of cancer. Especially, it can be utilized as a big data of cancer genome by using it as a method for analyzing the pathway of a specific gene variation and for detecting not only hot spot but also rare point mutation sensitively.

In this regard, about 10 driver mutations in lung adenocarcinoma are known, and several drugs targeting these variants have been reported. For example, gefitinib, which is an epithelial growth factor (EGFR) tyrosine kinase inhibitor, has a response rate of about 70% and patients with EGFR-activated mutations can survive for 10 months without tumor progression. Based on these studies, treatment strategies for lung adenocarcinoma have shifted from a tissue-based approach to a driver mutation-related therapy. The frequency of the occurrence of several driver mutations varies depending on ethnic groups, and thus for the development of new druggable targets and target therapies for lung cancer, broad genetic studies are needed.

Cancer cells with genetic modifications, called driver mutations, have advantages in survival and growth as compared to cells that do not have such modifications. Drive mutations occur in genes that encode signaling proteins such as kinase, which can generate survival signals that constitutively have activity of initiating and maintaining tumorigenesis.

As cancer is genetically changed very diversely and dynamically, it leads to a resistance to the treatment; after being progressed or metastasized, new mutations, which are different from a driver mutation that has played a key role at the time of diagnosis, may occur; or passenger mutations that were not significant at the time of diagnosis play a major role. Recently, as the concept of crisis model (chromothripsis), in which numerous multiple variations occur at once, unlike a multi-step model in which cancer occurs due to gradual stimulation and mutation, has been introduced, the evolution and heterogeneity of a tumor are the most difficult and important task to be solved in the future, and it is possible to identify various information by sensitively detecting various mutations using the present technology.

In addition, it can be utilized for monitoring the difference in gene expression according to individuals, various gene variations in cancer tissues of the primary cancer sites and metastasis sites and variations due to continuous evolution in the body of a patient, which are current limitations to the gene examination and utilization. In particular, the process of obtaining tissues from cancer patients is a difficult process that causes a great burden on the patient, while the present invention enables a method for detecting cancer-specific genes, which uses a small amount of tissue or from blood that is easy to be collected, so the present invention can be utilized as various data through various clinical demonstrations.

### Use for prevention, early diagnosis/diagnosis and prognosis of lung cancers and decision of a treatment method thereof

Lung cancer is a common cancer, which is known as the main cause of cancer-related deaths in the world. Although the rate of early diagnosis has been increased as low-dose computerized tomography screening techniques have been introduced, lung cancer is still a fatal disease with very poor prognosis.

If patients with lung cancer have an activation mutation of the epidermal growth factor receptor (EGFR) gene, EGFR-blocking tyrosine kinase inhibitors (e.g. Iressa, Tarceva, etc.) are effective in the treatment of lung cancer.

Among the existing EGFR-activated mutation test methods, a direct sequencing method is the standard method, and more sensitive PNA clamp method can be used. Also, it is possible to measure the mutation frequency in each patient tissue DNA by Ion Torrent analysis using the next generation sequencing method, along with the same sensitivity as the existing PNA clamp method, thereby obtaining more accurate information. In addition, there is a report that the therapeutic effect of EGFR-blocking tyrosine kinase inhibitor in patients with mutations detected in the sensitive method has higher clinical usability than the patient group in which the mutation is detected by direct sequencing method, so it is important to detect the genotype accurately and sensitively.

In case of a patient with lung cancer, the presence of at least one EGFR mutation indicates that the patient can have the benefit from treatment with gefitinib or erlotinib which is the EGFR-targeted compound. Abnormal activation or overexpression of EGFR has been reported in several types of cancer. Many studies have been conducted to find a target that plays a key role in the development of lung cancer and to understand genetic diversity, and it is possible to study genetic diversity using the present invention.

Oncogenes activated in lung cancer include EGFR, ALK, RAS, ROS1, MET, and RET, and therapeutic agents targeting these key oncogenes can selectively remove cancer cells. The epidermal growth factor receptor (EGFR), which is one of the ErbB tyrosine kinase receptors family (EGFR, HER-2, ErbB-3 and ErbB-4), is transmembrane tyrosine kinase which has an extracellular ligand-binding domain and an intra-cellular domain including a tyrosine kinase domain. When a ligand binds to a receptor forming homodimer or heterodimer, the tyrosine kinase in the cell is activated, and the signal stimulated by EGFR as such activates phosphatidylinositol 3-kinase (PI3K)/AKT/mTOR,RAS/RAF/MAPK, and JAK/STAT signaling pathways. Gefitinib and erlotinib are representative drugs that inhibit EGFR tyrosine kinase activity.

It has been discovered that mutations in the EGFR kinase domain are factors that can predict the effect of EGFR TKI, and these mutations are mainly expressed histologically in adenocarcinomas, females, nonsmokers, and Asians. Based on the results of several clinical trials, the EFGR mutation detection is conducted for patients diagnosed with stage IV adenocarcinoma of non-small-cell lung cancer, and if there is mutation, EGFR TKI is administered as a first-line treatment.

In addition, RAS proto-oncogene includes KRAS, NRAS, HRAS, etc. and regulates cell proliferation, differentiation and survival. RAS protein in the inactive state binds to guanosine diphosphate (GDP), and the RAS protein is activated when GDP is substituted with guanosine triphosphate (GTP), thereby activating RAS/RAF/MEK/MAPK and PI3K/AKT/mTOR signaling pathways. In lung cancer, activation of the RAS/RAF/MEK/MAPK signaling pathway occurs in approximately 20%, mainly due to KRAS mutation, and mutation of HRAS or NRAS is rare. Since KRAS plays an important role in the development of lung cancer, there have been many studies targeting it, but it has been reported that the studies using farnesyltransferase inhibitors that inhibit post translational processing of RAS proteins and antisense oligonucleotide to RAS have been unsuccessful. In addition, it has been reported that when KRAS mutation is present, the downstream of EGFR signaling pathway is continuously activated, thereby showing resistance to EGFR TKI. Thus, the present invention can be utilized to confirm this.

Meanwhile, as molecular biologic mechanisms that given the important influence on the development and progression of non-small cell lung cancer have been revealed, various diagnostic methods and target therapeutic agents have been developed, and as a result, the survival rate of patients with non-small cell lung cancer has been increased.

In particular, when EGFR tyrosine kinase inhibitor (TKI) is administered to an adenocarcinoma patient with an EGFR mutation, the response rate and progression-free survival time are significantly increased as compared to the conventional anticancer agents. In particular, crizotinib has attracted attention because it significantly reduces the time and expense for drug approval through patient selection with the accurate biomarker that predicts the response. Despite many studies, however, non-small cell lung cancer has a bad prognosis and has many problems to be solved in prevention, diagnosis and treatment. One of the causes is the genetic heterogeneity of lung cancer.

The results of the EGFR, KRAS and NRAS mutation-type test using the present invention can provide crucial information in the patient's treatment decision by conducting the test for the susceptibility of lung cancer drugs to predict the prognosis for the prescription of Gefitinib (Iressa) and Erlotinib (Tarceva), which are tyrosine kinase inhibitors (TKI) useful in the treatment of non-small cell lung cancer. In particular, the EGFR mutation is a marker that can accurately predict the response to EGFR-TKI, such as Gefitinib (Iressa) and Erlotinib (Tarceva), and the use thereof is recommended as a primary anti-cancer drug for EGFR mutation-positive non-small cell lung cancer patients.

In addition, the KRAS mutation acts as a strong predictor of drug resistance that does not respond to TKI drugs, and the KRAS mutation is mainly found in exon 2 codons 12 and 13. KRAS mutations are found in 15-30% of non-small cell lung cancer, mainly in patients with smoking history and are mutually exclusive with EGFR mutations.

Meanwhile, the therapeutic effect of a target therapeutic agent for lung cancer on non-small cell lung cancer patients has been improved by a chemotherapy in combination with vinorelbine, gemcitabine, taxanes and platinum, which are cytotoxic anticancer agents developed in the 1990s.

However, conventional cytotoxic anticancer agents were not greatly effective in the treatment of patients with progressive and metastatic non-small cell lung cancer. Since then, in molecular biology studies on the pathophysiology of lung cancer, gefitinib and erlotinib are used as secondary or tertiary therapeutic agents for monotherapy as a target therapeutic agent belonging to small molecule EGFR-TKI. In addition, recently, it has been confirmed that a triple therapy with an angiogenesis inhibitor, bevacizumab, in combination with the existing chemotherapeutic agent such as paclitaxel/carboplatin or gemcitabine/carboplatin, etc. has significance in the improvement of the survival time in the primary initial treatment of patients with progressive non-small cell lung cancer.

In the phase-2 study for patients with progressive non-small cell lung cancer who had a treatment history, gefitinib (Iressa) showed a response rate of 9-19% and symptomatic improvement in 40% or more of the patients, but in the large, randomized, placebo-controlled phase-3 study subsequently conducted, the addition of gefitinib to conventional paclitaxel/carboplatin cytotoxic anti-cancer therapies showed no significant differences in response rates, time to progression (TTP) and survival rates. In addition, according to the phase-4 study ISEL (Iressa Survival Evaluation in Lung Cancer) data recently reported, there was no significant difference in the survival rate between the gefitinib-treated group and the placebo-treated group. However, small group analysis showed differences in survival rates in nonsmokers and Asian populations. Currently, if a patient has an EGFR mutation or meets at least two of the three conditions such as female, adenocarcinoma, gefitinib is used as a secondary anti-cancer therapeutic agent, and gefitinib is used as a tertiary anti-cancer therapeutic agent in case of using taxanes and platinum in the primary and secondary anti-cancer treatment.

In addition, in the phase-2 study for patients with non-small cell lung cancer who had a treatment history, erlotinib (Tarceva) showed similar results to gefitinib with a response rate of 13% and a disease control rate of 51%, and patients with EGFR overexpression according to immunohistochemistry were subject to the treatment. However, the large phase-3 study, in which effects were observed by adding erlotinib to gemcitabine/cisplatin (TALENT) and carboplatin/paclitaxel (TRIBUTE) as a primary therapy, does not show significant improvement in response rate and survival period. However, in the result of BR.21 which is the placebo-controlled, 2:1 randomized phase-3 study, erlotinib, as a secondary therapy, showed in a significant difference in progression-free survival 2.2: 1.8 months and overall survival 6.7: 4.7 months, so erlotinib was reported as the first target therapeutic agent to demonstrate the survival data improvement in non-small cell lung cancer.

Recently, the study results that that anticancer agents are more effective than target therapeutic agents in lung cancer patients without EGFR mutations. In the treatment of patients with EGFR mutation-negative lung cancer, which accounts for about 60% of patients with lung cancer, conventional cytotoxic anticancer agents are more effective than "Iressa" and "Tarceva" which are the EGFR-targeted anti-cancer agents. In the clinical trial comparing EGFR-targeted anticancer agents with conventional anticancer agents and meta-analysis of patient treatment results, the studies were conducted to identify the best therapeutic agents for the EGFR mutation-negative patients who were subject to controversy about the efficacy of targeted anticancer agents, and as a result, EGFR mutation-negative patients, who were treated with conventional cytotoxic anticancer agents, showed a slower rate of cancer progression (median progression-free survival time 6.4 months versus 4.5 months), and decreased tumor size more (response rate 16.8% vs. 7.2%), compared to those treated with EGFR-targeted anticancer agents. These results were observed both when the anticancer agents were used as the primary therapeutic agent and when they were used as the secondary therapeutic agent.

Therefore, the results of mutation tests are important for future drug response tests, such as the recommendation that EGFR inhibitors should be preferentially used in patients with EGFR mutations.

### Use for prevention, early diagnosis/diagnosis and prognosis of colorectal cancers and decision of a treatment method thereof

In the meantime, regarding the cause of colorectal cancer identified so far, it has been reported that as genetic and epigenetic changes accumulate, transformation of a cell occurs, and tumor is developed due to the proliferation of transformed cells. Colorectal cancer is one of the tumors of which a genetic mechanism involved in the development of tumors is largely known. Recently, these genetic abnormalities have been applied to clinical trials and used in the treatment of patients. The present invention may be significant in the study of the mechanism of such cancer.

The oncogene, RAS, is present in three forms: HRAS, KRAS, and NRAS, and produces guanosine triphosphate hydrolase (GTPase), which functions as a molecular switch. RAS plays a role of delivering the extracellular growth signal into the nucleus, and when RAS is activated, RAS transforms cells through various pathways such as Raf/MAPK, PI3K/Akt, and Mekk/JNK, etc., and inhibits tumor suppressing pathways such as p53 or TGF-β pathway. Among RAS, KRAS mutations are commonly found in colorectal cancer, which occurs intensely at codons 12, 13, and 61, and it induces the resistance to GAP-mediated GTP hydrolysis, so it is continuously maintained in the activated state.

The fecal occult blood test for the diagnosis of colorectal cancer has been used for decades as a noninvasive colorectal cancer screening test and has been known as reducing the mortality rate of colorectal cancer in several studies (Mandel JS, N Engl J Med, 1993/ Kronborg O, Lancet 1996). However, there is a problem that sensitivity is low in screening colorectal cancer, especially precancerous conditions of colorectal cancer. In order to solve this problem, a fecal DNA test is performed. This test is to detect mutation or promoter hypermethylation occurring in colorectal cancer from feces.

In 2004, it was reported that the method of measuring mutations such as APC, KRAS, P53, etc. and 21 markers including BAT-26, long DNA, etc. with feces has an excellent sensitivity to colorectal cancer as compared to the fecal occult blood test (52% vs. 13%). In 2008, in the study using three markers such as mutations of KRAS, APC genes and promoter hypermethylation of vimentin gene, it was reported that the test showed progressive adenoma detection rate of 46% which is superior to the progressive adenoma detection rate of 10∼17% of fecal occult blood test (Imperiale TF N Engl J Med, 2004). This fecal DNA test has been known to have a sensitivity of 46-77% to early stage colorectal cancer and is thus superior to the fecal occult blood test, and recently it was added as colorectal cancer screening method. In addition, the studies to find DNA, RNA, proteins, etc. and use them for the screening test are in progress, and the present technology can be used to find candidates for screening tests or identifying the influence of specific mutations.

In addition, by demonstrating that in carcinogenesis, genetic instability and epigenetic changes play an important role and revealing various signaling pathways involved in the development of cancer, a new early diagnosis method using the genetic and epigenetic changes detected in colorectal cancer has been developed, and the method can be used as a predictor of prognosis and treatment response of colorectal cancer, together with the development of new medicines, thereby contributing to the development of personalized treatment.

### Use for prevention, early diagnosis/diagnosis and prognosis of colorectal cancers and decision of a treatment method thereof

Metastatic colorectal cancers (mCRC) are the second leading cause of death in the Western world.

Treatment based on monoclonal antibodies (moAb) against EGFR, for example, cetuximab and panitumumab provides survival benefit to patients with mCRC and is currently the standard component of therapy for these patients. That is, one of these moAbs, i.e. cetuximab (Erbitux), in combination with platinum-based chemotherapy, is also prescribed alone or in combination with other anti-tumor drug(s) for the treatment of patients with squamous cell carcinoma of the head and neck, called head and neck cancer.

The moAb binds to external antigens expressed on cancer cells. Once combined, the cancer cells are marked as destroyed by the immune system of the patient. In addition to targeting cancer cells, moAbs can be designed to act on other cell types and molecules needed for tumor growth. MoAb can be used to prevent tumor growth by destroying malignant tumor cells and blocking certain cellular receptors. Therapeutic moAb cetuximab and panitumumab bind to EGFR and prevent activation of intracellular signaling pathways (i.e. RAS-RAF-MEK-ERK cascade and PI3K-akt pathway) induced by EGFR. Unfortunately, not all patients with mCRC respond to treatment regimens involving moAbs. The mechanism underlying unresponsiveness is largely unknown.

The KRAS gene encoding the KRAS (Ki-ras2 Kirsten rat sarcoma viral oncogene homolog) protein can be derived from human, which is located on human chromosome 12 (p12.1), and the KRAS protein is a protein encoded thereby. The KRAS gene may have nucleotide sequence given to GenBank accession no. NM_004985, NM_033360, etc.

KRAS is an EGFR downstream effector and a marker of primary resistance to anti-EGFR moAb. KRAS has considerable influence on the optimization of treatment of mCRC patients. 40 percent of colorectal tumors carry mutations in the KRAS gene and these patients have no effect of anti-EGFR moAb. In the current clinical trials, all mCRC patients considered for anti-EGFR moAb therapy should undergo KRAS testing, and if a KRAS mutation is detected, the patient should be excluded from the treatment with cetuximab and panitumumab.

In addition, some of the mCRC patients with wild-type KRAS tumors still do not have benefit from anti-EGFR moAb. The rate of response to anti-EGFR moAb in wild-type KRAS subjects is approximately 60% when combined with chemotherapy and less than 20% when administered alone in chemotherapy-refractory patients.

Activating mutations of other EGFR downstream genes such as BRAF (serine/threonine-protein kinase B-Raf) and PI3K (phosphatidylinositol 3-kinase) as well as loss of expression of PTEN (phosphatase and tensin homologs), and the changes in other EGFR regulatory proteins have been evaluated as potent candidates for response to anti-EGFR therapies that have ever so far not conclusive results.

RAS proteins and RAF proteins are proteins that form a cascade of intracellular signal transduction by the RAS/RAF/MAPK pathway. In the RAS/RAF/MAPK pathway, the RAF protein is activated by the active RAS protein, the MEK protein is activated by the active RAF protein, and the MAPK protein is activated by the active MEK protein. As a result, they regulate the proliferation and differentiation of cells.

Bevacizumab/FOLFIRI combination therapy is an appropriate treatment as a primary therapy for metastatic colorectal cancer and can prolong the response rate, progression-free survival, and overall survival (Hurwitz H, et al. NEJM June, 2004, Petrelli et al. Clin Colorectal Cancer. 2013 Sep; 12 (3): 145-51). The therapeutic agent with efficacy for progression-free survival and overall survival as a secondary therapy for metastatic intestinal colorectal cancer is bevacizumab and ziv-aflibercept (Giantonio et al. JCO 2007 14. Tabernero et al. European Journal of Cancer 50, 320-331, 2014).

When bevacizumab was administered in combination with chemotherapy in the primary treatment, and after first recurrence, bevacizumab is continuously administered with changed chemotherapy in the secondary therapy, progression-free survival and overall survival were statistically and significantly prolonged (Bennouna et al. Lancet Oncol. 14: 29: 37, 2013).

### [Examples]

### [Example 1]

### EGFR Mutation Detection in Patients with Non-small cell Lung Cancer

### Example 1-1. Sample collection

Tissue samples from 192 non-small cell lung cancer patients participating in the randomized phase 2 study and plasma samples isolated from the blood were obtained. For the tissue samples, the results were obtained using the sequencing method, and the EGFR test was performed from the plasma using the method for simultaneous detection of multiple target nucleic acids (C-Melting technology) according to the present invention.

### Example 1-2. DNA extraction

For this experiment, DNA was extracted using QIAamp® Circulating Nucleic Acid Kit (Cat # 55114) from QIAGEN. In summary, 100 µl of Proteinase K and 800 µl of a buffered ACL containing 1.0 µg of carrier RNA were added to 1 ml of a plasma sample and mixed well, and then treated at 60°C for one hour. After culturing it, 1.8 ml of ACB buffer was added and the mixture remained on ice for 5 minutes. After the solution was loaded on a column using vacuum, the column was washed with 600 µl of buffer ACW1, 750 µl of buffer ACW2 and 750 µl of 100% ethanol in order. The column was separated from the vacuum system, centrifuged at 14,000 rpm for 3 minutes, and then treated at 56°C for 10 minutes to remove residual ethanol. 100 µl of buffer AVE was added to the column and centrifuged at 14,000 rpm for 1 minute to elute the DNA. 5 µl of the eluted DNA was used in C-melting experiment.

### Example 1-3. EGFR mutation confirmation

PCR reaction was performed to detect EGFR mutation using DNA extracted from plasma. In summary, 1 µl of Taq DNA polymerase (Enzynomics., Korea) was mixed with 19 µl of each of 10 reagents (G719X, E19del A, E19del B, S768I, T790M, E20ins A, E20ins B, L858R, L861Q, EIC) for the detection of 47 mutations of exons 18, 19, 20 and 21, and then 5 µl of DNA was added. PCR was carried out for 2 minutes at 50°C for inactivation of UDG, followed by 15 cycles (95°C for 30 seconds, 70°C for 20 seconds, and 63°C for 1 minute) and 35 cycles (95°C for 10 seconds, 53°C for 20 seconds, 73°C for 20 seconds) after initial denaturation at 95°C for 15 minutes. Then, the cells were cultured at 95°C for 15 minutes and at 35°C for 5 minutes, and the signals (FAM, HEX, ROX, Cy5) were measured every 3 seconds while increasing the temperature from 35°C to 75°C by 0.5°C to derive the Tm value. The PCR reaction was applied to all DNAs extracted from plasmas of 192 patients.

### Example 1-4. Correlation with tissue results and survival analysis

Using the C-melting technique of the present invention, the EGFR test results of the DNA extracted from plasma samples showed the consistency rate of 81.3% (95% confidence interval [CI], 75.0-86.5) with existing tissue samples, sensitivity and specificity of 62.0% (95% CI, 47.2-75.4) and 88.0% (95% CI, 81.5-92.9), respectively. In addition, EGFR T790M (n = 8) and exon 20ins (n = 5) were additionally detected in plasma samples as compared to tissue samples. Table 1 shows the median progression-free survival (PFS) results among 114 patients treated with EGFR-TKI (Gefitinib (Iressa) or Erlotinib (Tarceva)).

### Table 1

**[Table 1]**

| | Average PFS (mo) | P |
|---|---|---|
| Tissue EGFR 19del or L858R pos vs. neg | 9.2 vs 1.7 | < .0001 |
| Plasma EGFR 19del or L858R pos vs. neg | 8.4 vs 1.9 | .045 |

As a result of analyzing the progression-free survival time based on the EGFR mutation detection result using the method of the present invention, significant survival results were confirmed.

### [Example 2]

### KRAS Mutation Detection in Patients with Non-small cell Lung Cancer

### Example 2-1. Sample collection

Tissue samples from 135 non-small cell lung cancer patients participating in the randomized phase 2 study and plasma samples isolated from blood were obtained. For tissue samples, results were obtained using the sequencing method, and the KRAS test was performed from the plasma using the method for simultaneous detection of multiple target nucleic acids (C-Melting technology) according to the present invention.

### Example 2-2. DNA extraction

For this experiment, DNA was extracted using QIAamp® Circulating Nucleic Acid Kit (Cat # 55114) from QIAGEN. In summary, 100 µl of Proteinase K (QIAGEN) and 800 µl of a buffered ACL containing 1.0 µg of carrier RNA were added to 1 ml of a plasma sample and mixed well, and then treated at 60°C for one hour. After culturing it, 1.8 ml of ACB buffer was added and the mixture remained on ice for 5 minutes. After the solution was loaded on a column using vacuum, the column was washed 600 µl of buffer ACW1, 750 µl of buffer ACW2 and 750 µl of 100% ethanol in order. The column was separated from the vacuum system, centrifuged at 14,000 rpm for 3 minutes, and then treated at 56°C for 10 minutes to remove residual ethanol. 100 µl of buffer AVE was added to the column and centrifuged at 14,000 rpm for 1 minute to elute the DNA. 5 µl of the eluted DNA was used in C-melting experiment.

### Example 2-3. KRAS mutation confirmation

PCR reaction was performed to detect KRAS mutation using DNA extracted from plasma. In summary, 1 µl of Taq DNA polymerase (Enzynomics., Korea) was mixed with 19 µl of each of 9 reagents (KC12a, KC12b, KC13a, KC13b, KC59, KC61, KC117, KC146 and KIC) for the detection of 29 mutations of exons 2, 3 and 4, and then 5 µl of DNA was added. PCR was carried out for 2 minutes at 50°C for inactivation of UDG, followed by 15 cycles (95°C for 30 seconds, 70°C for 20 seconds, and 63°C for 1 minute) and 35 cycles (95°C for 10 seconds, 53°C for 20 seconds, 73°C for 20 seconds) after initial denaturation at 95°C for 15 minutes. Then, the cells were cultured at 95°C for 15 minutes and at 35°C for 5 minutes, and the signals (FAM, HEX, ROX, Cy5) were measured every 3 seconds while increasing the temperature from 35°C to 75°C by 0.5°C to derive the Tm value. The PCR reaction was applied to all DNAs extracted from plasmas of 135 patients.

### Example 2-4. Correlation with tissue results and survival analysis

Using the C-melting technique of the present invention, the KRAS test results of the DNA extracted from plasma samples showed the consistency rate of 81.5% (95% confidence interval [CI], 73.9-87.6) with existing tissue samples, sensitivity and specificity of 25.0% (95% CI, 5.5-57.2) and 87.0% (95% CI, 79.7-92.4), respectively. In addition, a mutation was additionally detected in samples of 16 patients in the plasma sample as compared to the tissue sample. Table 2 shows the median progression-free survival (PFS) results for 114 patients treated with EGFR-TKI.

### Table 2

**[Table 2]**

| | Average PFS (mo) | P |
|---|---|---|
| Tissue KRAS pos vs. neg | 1.8 vs 5.4 | .073 |
| Plasma KRAS pos. vo. neg | 1.6 vs. 5.5 | 0.003 |

As a result of analyzing the progression-free survival time based on the KRAS mutation test results using the method of the present invention, significant survival results were confirmed.

## Claims

1. A method for detecting mutations of EGFR or KRAS in the sample from patients to provide information necessary for the treatment with administration of Tyrosine Kinase Inhibitor (TKI) for a cancer disease, comprising:
(a) performing hybridization by mixing a primer and a probe mixture composed of at least one detection probe for detecting mutants of EGFR or KRAS and a clamping probe for inhibiting amplification of wild-type EGFR gene or wild-type KRAS gene in the sample from patients, and obtaining a real-time amplification curve;
(b) obtaining a dissociation curve between an amplified product and the detection probe with changing temperature after the amplification; and
(c) analyzing the obtained real-time amplification curve and dissociation curve separately, sequentially or simultaneously,
wherein the detection probe and the clamping probe is peptide nucleic acid (PNA), and
an amino acid or a side chain of an amino acid is linked to the gamma position of the detection probe and the clamping probe for structural modification.

2. The method according to claim 1, **characterized in that** the Tyrosine Kinase Inhibitor (TKI) is Cetuximab or Panitumumab.

3. The method according to claim 1 or 2, **characterized in that** the cancer disease is selected from the group consisting of lung cancer, ovarian cancer, cervical cancer, endometrial cancer, breast cancer, brain cancer, colon cancer, prostate cancer, gastrointestinal cancer, head and neck cancer, nonsmall-cell lung cancer, nervous system cancer, renal cancer, retina cancer, skin cancer, liver cancer, pancreatic cancer, genital-urinary tract cancer, gallbladder cancer, melanoma, and leukemia.

4. The method according to claim 1 or 2, **characterized in that** the sample from patients is selected from the group consisting of blood, serum, plasma, lymph, milk, urine, feces, eye fluid, saliva, semen, brain extract, spinal fluid, and extracts from appendix, spleen, and tonsil.

5. The method according to claim 1 or 2, **characterized in that** a reporter and a quencher is linked to the detection probe.

6. The method according to claim 1 or 2, **characterized in that** the amplification is performed by a real-time polymerase chain reaction (PCR).

7. The method according to claim 1 or 2, **characterized in that** 5 to 20 cycles of PCR reaction are added prior to obtaining the dissociation curve in (b), separately from obtaining the real-time amplification curve.

8. The method according to claim 1, **characterized in that** the Tyrosine Kinase Inhibitor (TKI) is Gefitinib or Erlotinib.

9. Use of a kit for detecting EGFR or KRAS using the method according to claim 1 or 2.

## Patentansprüche

1. Ein Verfahren zum Nachweis von Mutationen von EGFR oder KRAS in der Probe von Patienten, um Informationen bereitzustellen, die für die Behandlung mit der Verabreichung von Tyrosinkinase-Inhibitor (TKI) für eine Krebserkrankung erforderlich sind, umfassend:
(a) Durchführen einer Hybridisierung durch Mischen einer Primer- und einer Sondenmischung, die aus mindestens einer Nachweissonde zum Nachweis von Mutanten von EGFR oder KRAS und einer Klammersonde zur Hemmung der Amplifikation des Wildtyp-EGFR-Gens oder des Wildtyp-KRAS-Gens in der Probe von Patienten besteht, und Erhalten einer Echtzeit-Amplifikationskurve;
(b) Erhalten einer Dissoziationskurve zwischen einem amplifizierten Produkt und der Nachweissonde mit Temperaturänderung nach der Amplifikation; und
(c) getrenntes Analysieren der erhaltenen Echtzeit-Amplifikationskurve und Dissoziationskurve, sequentiell oder gleichzeitig,
wobei die Detektionssonde und die Klammersonde Peptid-Nukleinsäure (PNA) ist, und
eine Aminosäure oder eine Seitenkette einer Aminosäure mit der Gamma-Position der Detektionssonde und der Klammersonde zur Strukturmodifikation verbunden ist.

2. Das Verfahren nach Anspruch 1, **dadurch gekennzeichnet, dass** der Tyrosinkinase-Inhibitor (TKI) Cetuximab oder Panitumumab ist.

3. Das Verfahren nach Anspruch 1 oder 2, **dadurch gekennzeichnet, dass** die Krebserkrankung ausgewählt wird aus der Gruppe bestehend aus Lungenkrebs, Eierstockkrebs, Gebärmutterhalskrebs, Endometriumkrebs, Brustkrebs, Hirnkrebs, Dickdarmkrebs, Prostatakrebs, Magen-Darm-Krebs, Kopf- und Halskrebs, nichtkleinzelligem Lungenkrebs, Krebs des Nervensystems, Nierenkrebs, Retinakrebs, Hautkrebs, Leberkrebs, Bauchspeicheldrüsenkrebs, Krebs des Genital-Harntrakts, Gallenblasenkrebs, Melanom und Leukämie.

4. Das Verfahren nach Anspruch 1 oder 2, **dadurch gekennzeichnet, dass** die Probe von Patienten ausgewählt wird aus der Gruppe bestehend aus Blut, Serum, Plasma, Lymphe, Milch, Urin, Kot, Augenflüssigkeit, Speichel, Sperma, Himextrakt, Rückenmarksflüssigkeit und Extrakten aus Blinddarm, Milz und Mandeln.

5. Das Verfahren nach Anspruch 1 oder 2, **dadurch gekennzeichnet, dass** ein Reporter und ein Quencher mit der Nachweissonde verbunden ist.

6. Das Verfahren nach Anspruch 1 oder 2, **dadurch gekennzeichnet, dass** die Amplifikation durch eine Echtzeit-Polymerase-Kettenreaktion (PCR) durchgeführt wird.

7. Das Verfahren nach Anspruch 1 oder 2, **dadurch gekennzeichnet, dass** 5 bis 20 Zyklen der PCR-Reaktion vor dem Erhalt der Dissoziationskurve in (b) hinzugefügt werden, getrennt vom Erhalt der Echtzeit-Amplifikationskurve.

8. Das Verfahren nach Anspruch 1, **dadurch gekennzeichnet, dass** der Tyrosinkinase-Inhibitor (TKI) Gefitinib oder Erlotinib ist.

9. Verwendung eines Kits zum Nachweis von EGFR oder KRAS unter Verwendung des Verfahrens nach Anspruch 1 oder 2.

## Revendications

1. Procédé de détection de mutations d'EGFR ou de KRAS dans l'échantillon issu de patients pour fournir des informations nécessaires pour le traitement avec administration d'inhibiteur de tyrosine kinase (TKI) pour une maladie cancéreuse, comprenant :
(a) l'exécution d'une hybridation en mélangeant une amorce et un mélange de sondes composé d'au moins une sonde de détection destinée à détecter des mutants d'EGFR ou de KRAS et une sonde de blocage destinée à inhiber l'amplification de gène EGFR de type sauvage ou de gène KRAS de type sauvage dans l'échantillon issu de patients, et l'obtention d'une courbe d'amplification en temps réel ;
(b) l'obtention d'une courbe de dissociation entre un produit amplifié et la sonde de détection avec une température changeante après l'amplification; et
(c) l'analyse, séparément, séquentiellement ou simultanément, de la courbe d'amplification en temps réel et de la courbe de dissociation obtenues,
sachant que la sonde de détection et la sonde de blocage sont de l'acide nucléique de peptide (PNA), et un acide aminé ou une chaîne latérale d'un acide aminé est lié à la position gamma de la sonde de détection et de la sonde de blocage pour modification structurelle.

2. Le procédé selon la revendication 1, **caractérisé en ce que** l'inhibiteur de tyrosine kinase (TKI) est du cetuximab ou du panitumumab.

3. Le procédé selon la revendication 1 ou 2, **caractérisé en ce que** la maladie cancéreuse est sélectionnée dans le groupe constitué par le cancer du poumon, le cancer de l'ovaire, le cancer du col de l'utérus, le cancer de l'endomètre, le cancer du sein, le cancer du cerveau, le cancer du colon, le cancer de la prostate, le cancer gastrointestinal, le cancer du cou et de la tête, le cancer du poumon non à petites cellules, le cancer du système nerveux, le cancer du rein, le cancer de la rétine, le cancer de la peau, le cancer du foie, le cancer du pancréas, le cancer des parties génitales et du tractus urinaire, le cancer de la vésicule biliaire, le mélanome, et la leucémie.

4. Le procédé selon la revendication 1 ou 2, **caractérisé en ce que** l'échantillon issu de patients est sélectionné dans le groupe constitué par le sang, le sérum, le plasma, la lymphe, le lait, l'urine, les selles, le liquide oculaire, la salive, le semen, l'extrait de cerveau, le fluide spinal, et les extraits d'appendice, de rate, et de tonsille.

5. Le procédé selon la revendication 1 ou 2, **caractérisé en ce qu'**un rapporteur et un quencher sont liés à la sonde détection.

6. Le procédé selon la revendication 1 ou 2, **caractérisé en ce que** l'amplification est effectuée par une réaction en chaîne par polymérase (PCR) en temps réel.

7. Le procédé selon la revendication 1 ou 2, **caractérisé en ce que** 5 à 20 cycles de réaction PCR sont ajoutés avant l'obtention de la courbe de dissociation en (b), séparément de l'obtention de la courbe d'amplification en temps réel.

8. Le procédé selon la revendication 1, **caractérisé en ce que** l'inhibiteur de tyrosine kinase (TKI) est du gefitinib ou de l'erlotinib.

9. Utilisation d'un kit pour la détection d'EGFR ou de KRAS moyennant le procédé selon la revendication 1 ou 2.
